# EUROPEAN PATENT APPLICATION

(11) **EP 1 731 609 A1**
(43) Date of publication of application: **13.12.2006**
(21) Application number: 05727900.2
(22) Date of filing: 30.03.2005
(51) Int. Cl.: C12N 15/12, A61K 31/282, A61K 31/7088, A61K 33/24, A61K 35/76, A61K 38/00, A61K 45/00, A61K 48/00, A61P 35/00, A61P 43/00

(54) **Rad51 EXPRESSION INHIBITOR, DRUG CONTAINING THE INHIBITOR AS THE ACTIVE INGREDIENT AND UTILIZATION THEREOF**

(30) Priority: 30.03.2004 JP 2004098839
(71) Applicant: GenomIdea Inc., Osaka 567-0085 (JP)
(72) Inventor: KANEDA, Yasufumi, Minoo-shi, Osaka 5620031 (JP)
(74) Representative: Grünecker, Kinkeldey, Stockmair & Schwanhäusser Anwaltssozietät
(86) International application number: PCT/JP2005/006075
(87) International publication number: WO 2005/095613

(57) **Abstract**

A double stranded RNA having a specific nucleotide sequence is useful for treating cellular proliferative disorders. The double stranded RNA acts as siRNA against the Rad51 gene. The double stranded RNA of the present invention inhibits cellular proliferation itself. In addition, co-administration of the double stranded RNA with a chemotherapeutic agent further enhances its pharmacological effect. The combined use of the double stranded RNA with an agent having a nucleic acid synthesis-inhibitory activity or a nucleic acid-impairing activity is particularly effective.

## Description

### Technical Field

The present invention relates to Rad51 expression inhibitors, pharmaceutical agents that include the inhibitors as active ingredients, methods for preparing the pharmaceutical agents, and methods for treating cancers or malignant tumors.

### Background Art

For the last decade, cancer has been the leading cause of death in Japan. Cancer cells can be explained as the cells that have developed a defect in the cell division-regulatory mechanism and started an unlimited division and proliferation. Cancer is caused by such uncontrolled proliferation of cancer cells, which in turn impairs normal tissues and their functions.

Cancer therapies include physico-scientific treatment, surgical treatment, and drug treatment. Physico-scientific treatment involves exposing the cancerous lesion to radiation or proton beam so that cancer cells will be killed by the cytotoxic activities of such radiation. Surgical treatment involves surgical operations to excise solid tumors. Drug treatment involves administering an agent which inhibits the survival or proliferation of cancer cells.

Cancer therapy that involves administering an agent containing a chemically synthesized compound or a compound isolated from non-human organisms as an active ingredient is generally referred to as "chemotherapy". Agents used for chemotherapy are referred to as chemotherapeutic agents. As detailed below, chemotherapeutic agents can be classified based on their mode of action.

Carcinostatic agents: Carcinostatic agents are agents that inhibit cancer cell proliferation. Since uncontrolled proliferation of cancer cells causes disorders, these agents, capable of inhibiting the proliferation of cancer cells, are expected to have therapeutic effects, even if they do not result in the destruction of the cancer cell itself.

Anticancer agents: While the carcinostatic agents inhibit cancer cell proliferation, agents having cancer cytotoxic or cell-killing activities are specially termed anticancer agents. Although the carcinostatic agents inhibit cancer progression, they have little effect with regard to providing cancer regression. In contrast, the anticancer agents can be expected to have cancer regression effects. The anticancer agent is a subcategory of the carcinostatic agent.

Antitumor agents or antineoplastic agents: While the carcinostatic agents and anticancer agents refer to agents having an action against cancer or malignant tumor, antitumor agents refer to agents having an action against all tumors, including benign tumors. Antitumor agents can also be described as agents that suppress proliferative cells.

Cancer treatment by drug administration also includes administration of the so-called cancer vaccine or immunopotentiator that induces an antitumor immunity in a host. Cancer treatment by drug administration further includes administration of an antibody that attacks cancer, or a cancer-binding antibody labeled with a cytotoxic compound. From these methods of treatment, an appropriate treatment is selected in consideration of the cancer type, cancer stage, host condition, etc. In general, these therapeutic methods are used in combination.

More recently, cancer therapy involving the regulation of expression of a gene associated with cancer development or proliferation has been applied to clinical settings. For example, the following genes are known for their use in cancer therapy through inhibition of their expression:
- c-myc;
- H-ras; and
- c-raf kinase. Antisense and ribozyme technologies have been used for the inhibition of the gene expression.

In addition, it has been reported that the efficacy of cancer therapy can be enhanced by inhibiting the cell-repairing mechanism of cancer. Cells have mechanisms to repair DNA damage caused by ionizing radiation or certain anticancer agents. For example, cleaved double stranded DNA may be repaired through a homologous or non-homologous recombination repair mechanism. In the homologous recombination repair mechanism, the cleaved double strand is repaired by recombination using a homologous chromosome or a sister-chromatid as a template. The following enzymes are known to be required for the homologous recombination repair: Rad51, Rad52, Rad54, Rad51B, Rad51C, Rad51D, XRCC2, and XRCC3. Among them, Rad51 is known for its utility in enhancing radiosensitivity of tumor when its expression is suppressed by an antisense oligonucleotide (US 2003/0229004, WO 03/13488). It is also known that inhibition of RAD51 enhances the effect of gene therapy using p53 (WO 02/58738).

Recently, RNA interference (RNAi) has been gaining attention as a gene expression-regulatory technology. RNAi is a phenomenon where a double stranded RNA having the same nucleotide sequence as mRNA cleaves the mRNA, resulting in inhibition of protein expression (Nature, 1998 Feb 19; 391 (6669): 806-11; Nature, 2001 May 24; 411 (6836): 494-8). It has been reported that the expression-inhibitory effect of RNAi is 10³- to 10⁷-fold higher than that of antisense oligonucleotides, another gene expression-regulatory technology (Biochem. Biophys. Res. Commun. 296: 1000-1004, 2002). The double stranded RNA having the RNAi effect is specifically called siRNA (small interfering RNA). siRNA has been shown to be effective in cancer therapy using, for example, the following genes whose mutations are known to be responsible for carcinogenesis:
- k-ras (Nucleic Acids Res., 31: 700-707, 2003); and
- p53 (Proc. Natl. Acad. Sci. USA., 99: 14849-14854, 2002).

Patent Document 1: US 2003/0229004
Patent Document 2: WO 03/13488
Patent Document 3: WO 02/58738
Non-Patent Document 1: Fire A. et al., Nature, 1998 Feb 19; 391 (6669): 806-11
Non-Patent Document 2: Elbashir SM. et al., Nature, 2001 May 24; 411 (6836): 494-8
Non-Patent Document 3: Biochem. Biophys. Res. Commun. 296: 1000-1004, 2002
Non-Patent Document 4: Nucleic Acids Res., 31: 700-707, 2003
Non-Patent Document 5: Martinez LA et al., Proc. Natl. Acad. Sci. USA., 99: 14849-14854, 2002

### Summary of the Invention

### Problems to Be Solved by the Invention

An objective of the present invention is to provide a therapeutic technology for cell proliferative disorders. More specifically, an objective of the present invention is to provide a therapeutic technology for cancer through the regulation of Rad51 expression.

### Means to Solve the Problems

Rad51 is a mammalian homolog of *E. coli* RecA. It has been reported that Rad51-knockout mice are embryonic lethal. This information strongly suggests that Rad51 plays a very important role *in vivo.* Moreover, it is known that inhibiting the Rad51 expression using an antisense oligonucleotide enhances the radiosensitivity of tumors.

The inventors of the present invention considered that the inhibition of Rad51 expression using siRNA would be useful for tumor therapy, and thus searched for an effective siRNA. As a result, the inventors discovered that double stranded RNA including the nucleotide sequence of SEQ ID NO: 1 is effective as siRNA against Rad51. Moreover, the inventors of the present invention revealed that the combined use of the siRNA and a chemotherapeutic agent exhibits a significant inhibitory effect on cell proliferation. The present inventors also found that viral envelope vectors are advantageous in the administration of siRNA or compositions containing siRNA and a chemotherapeutic agent. Specifically, the present invention relates to the following pharmaceutical compositions, double stranded RNA useful as siRNA, and uses thereof.
[1] A double stranded RNA composed of a sense strand comprising the nucleotide sequence of SEQ ID NO: 1 and a complementary strand thereof, wherein said sense strand is 100 nucleotides or less in length.
[2] A vector expressing a double stranded RNA composed of a sense strand comprising the nucleotide sequence of SEQ ID NO: 1 and a complementary strand thereof, wherein said sense strand is 100 nucleotides or less in length.
[3] An inhibitor of the expression of the Rad51 gene comprising the double stranded RNA of [1] or the vector of [2] as an active ingredient.
[4] The inhibitor of [3], wherein said Rad51 gene is a human Rad51 gene.
[5] A pharmaceutical composition comprising a double stranded RNA composed of a sense strand comprising the nucleotide sequence of SEQ ID NO: 1 and a complementary strand thereof, or a vector capable of expressing the RNA, as an active ingredient.
[6] A pharmaceutical composition comprising a double stranded RNA composed of a sense strand comprising the nucleotide sequence of SEQ ID NO: 1 and a complementary strand thereof, or a vector capable of expressing the RNA, and a chemotherapeutic agent as active ingredients.
[7] The pharmaceutical composition of [6], wherein said pharmaceutical composition is used to treat a cancer or malignant tumor.
[8] The pharmaceutical composition of [6], wherein said chemotherapeutic agent is selected from the group consisting of carcinostatic agents, anticancer agents, antitumor agents, and antineoplastic agents.
[9] The pharmaceutical composition of [6], wherein said chemotherapeutic agent has either a nucleic acid synthesis inhibitory activity or a nucleic acid impairing activity, or both.
[10] The pharmaceutical composition of [6], wherein said chemotherapeutic agent is at least one compound selected from the group consisting of bleomycins, anthraquinone series carcinostatic agents, mitomycins, actinomycins, camptothecins, cisplatins, streptozotocin, 5-fluorouracil (5-FU) and derivatives thereof, pirarubicin, and pharmaceutically acceptable salts thereof.
[11] The pharmaceutical composition of [10], wherein said bleomycins comprise a compound selected from the group consisting of bleomycin and peplomycin.
[12] The pharmaceutical composition of [10], wherein said pharmaceutically acceptable salts of bleomycins comprise a compound selected from the group consisting of bleomycin hydrochloride, bleomycin sulfate, and peplomycin sulfate.
[13] The pharmaceutical composition of [10], wherein said cisplatins comprise a compound selected from the group consisting of cisplatin, paraplatin, and briplatin.
[14] The pharmaceutical composition of [6], wherein said chemotherapeutic agent is encapsulated in a viral envelope vector together with the double stranded RNA.
[15] The pharmaceutical composition of [14], wherein said viral envelope vector is composed of an envelope derived from a virus belong to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae.
[16] The pharmaceutical composition of [14], wherein said viral envelope vector is composed of an envelope derived from a virus selected from the group consisting of Sendai virus, retrovirus, adenovirus, adeno-associated virus, Herpes virus, vaccinia virus, poxvirus, and influenza virus.
[17] A combined composition comprising (a) a pharmaceutical composition comprising a double stranded RNA composed of a sense strand comprising the nucleotide sequence of SEQ ID NO: 1 and a complementary strand thereof as an active ingredient, and (b) a pharmaceutical composition comprising a chemotherapeutic agent as an active ingredient.
[18] A combined composition comprising (a) a pharmaceutical composition comprising a vector capable of expressing a double stranded RNA composed of a sense strand comprising the nucleotide sequence of SEQ ID NO: 1 and a complementary strand thereof as an active ingredient, and (b) a pharmaceutical composition comprising a chemotherapeutic agent as an active ingredient.
[19] The combined composition of [17] or [18], wherein said combined composition is used to treat a cancer or malignant tumor.
[20] The combined composition of any one of [17] to [19], wherein said chemotherapeutic agent is selected from the group consisting of carcinostatic agents, anticancer agents, antitumor agents, and antineoplastic agents.
[21] The combined composition of any one of [17] to [19], wherein said chemotherapeutic agent has either a nucleic acid synthesis inhibitory activity or a nucleic acid impairing activity, or both.
[22] The combined composition of any one of [17] to [19], wherein said chemotherapeutic agent is at least one compound selected from the group consisting of bleomycins, anthraquinone series carcinostatic agents, mitomycins, actinomycins, camptothecins, cisplatins, streptozotocin, 5-fluorouracil (5-FU) and derivatives thereof, pirarubicin, and pharmaceutically acceptable salts thereof.
[23] The combined composition of [22], wherein said bleomycins comprise a compound selected from the group consisting of bleomycin and peplomycin.
[24] The combined composition of [22], wherein said pharmaceutically acceptable salts of bleomycins comprise a compound selected from the group consisting of bleomycin hydrochloride, bleomycin sulfate, and peplomycin sulfate.
[25] The combined composition of [22], wherein said cisplatins comprise a compound selected from the group consisting of cisplatin, paraplatin, and briplatin.
[26] The combined composition of [17] or [18], wherein said double stranded RNA is encapsulated in a viral envelope vector.
[27] The combined composition of [26], wherein said viral envelope vector is composed of an envelope derived from a virus belong to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae.
[28] The combined composition of [26], wherein said viral envelope vector is composed of an envelope derived from a virus selected from the group consisting of Sendai virus, retrovirus, adenovirus, adeno-associated virus, Herpes virus, vaccinia virus, poxvirus, and influenza virus.
[29] A method for inhibiting Rad51 expression, comprising the step of administering into cells the double stranded RNA of [1] or the vector of [2].
[30] A method for treating a cancer or malignant tumor, comprising the step of administering a double stranded RNA composed of a sense strand comprising the nucleotide sequence of SEQ ID NO: 1 and a complementary strand thereof, or a vector capable of expressing the RNA, and a chemotherapeutic agent.
[31] The method of [30], wherein said step of administering the double stranded RNA or the vector capable of expressing the RNA and the chemotherapeutic agent comprises administering a viral envelope vector in which the double stranded RNA and the chemotherapeutic agent have been encapsulated.
[32] The method of [30], wherein said step of administering the double stranded RNA or the vector capable of expressing the RNA and the chemotherapeutic agent comprises separately administering a pharmaceutical composition comprising the double stranded RNA or the vector capable of expressing the RNA as an active ingredient, and a pharmaceutical composition comprising the chemotherapeutic agent as an active ingredient.
[33] Use of the double stranded RNA of [1] or the vector of [2] for preparing a pharmaceutical composition for treating a cancer or malignant tumor.
[34] Use of the double stranded RNA of [1] or the vector of [2], wherein said pharmaceutical composition comprises a chemotherapeutic agent.
[35] A pharmaceutical composition comprising bleomycin or a pharmaceutically acceptable salt thereof and a Rad51 inhibitor.
[36] A pharmaceutical composition comprising cisplatin or a pharmaceutically acceptable salt thereof and a Rad51 inhibitor.
[37] A combined composition comprising (a) a pharmaceutical composition comprising bleomycin or a pharmaceutically acceptable salt thereof as an active ingredient, and (b) a pharmaceutical composition comprising a Rad51 inhibitor as an active ingredient.
[38] A combined composition comprising (a) a pharmaceutical composition comprising cisplatin or a pharmaceutically acceptable salt thereof as an active ingredient, and (b) a pharmaceutical composition comprising a Rad51 inhibitor as an active ingredient.
[39] The combined composition of [37] or [38], wherein said combined composition is intended to treat a cancer or malignant tumor.

### Effects of the Invention

The present invention provides double stranded RNA useful as siRNA against Rad51. The double stranded RNA of the present invention effectively suppresses the expression of Rad51. The double stranded RNA of the present invention is useful as an inhibitor of cancer cell proliferation. The double stranded RNA of the present invention is also effective in enhancing the therapeutic effect of radiation cancer therapy. An antisense oligonucleotide against Rad51 has been known to enhance the radiosensitivity of cancer. The double stranded RNA of the present invention suppresses the gene expression much more strongly than the antisense oligonucleotide. Thus, the double stranded RNA is expected to have a stronger radiosensitivity-enhancing effect.

In addition, the double stranded RNA of the present invention exhibits more significant carcinostatic activity when combined with a chemotherapeutic agent. Thus, the present invention provides pharmaceutical compositions containing the double stranded RNA and a chemotherapeutic agent. The pharmaceutical compositions of the present invention are useful in treating cancer.

### Brief Description of the Drawings

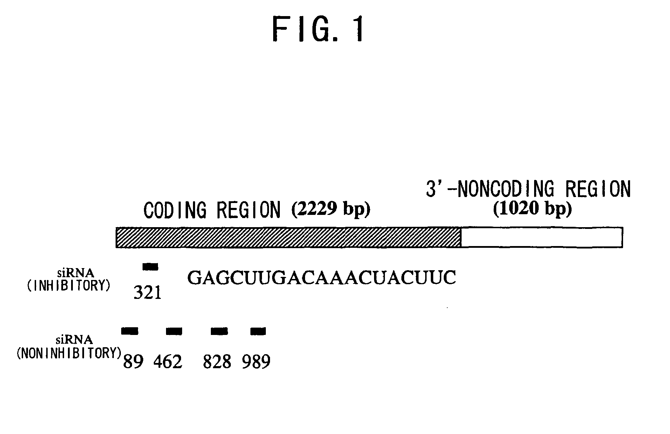

Fig. 1 depicts the targeting sites of five Rad51 siRNAs. siRNA 321, 462, 989, 89, and 828 correspond to SEQ ID NOs: 1, 2, 3, 4, and 5, respectively. The expression inhibitory effect was found only with siRNA 321 (SEQ ID NO: 1); the other siRNAs were ineffective.

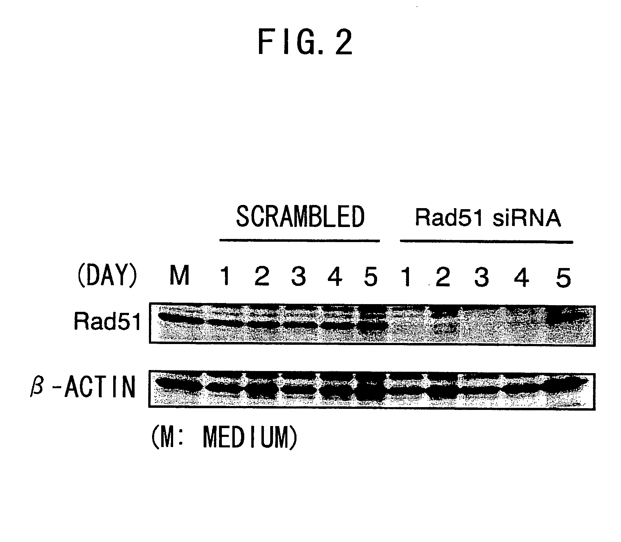

Fig.2 depicts the knockdown of Rad51 using siRNA. Rad51 siRNA (target 321) was introduced into HeLa cells using an HVJ envelope vector, followed by monitoring the Rad51 protein expression on a day-by-day basis. As a control, a scrambled siRNA (SEQ ID NO: 6) was used. β-actin was also detected as an internal protein standard. This figure shows that Rad51 siRNA (321) is capable of inhibiting the Rad51 protein expression for a long time.

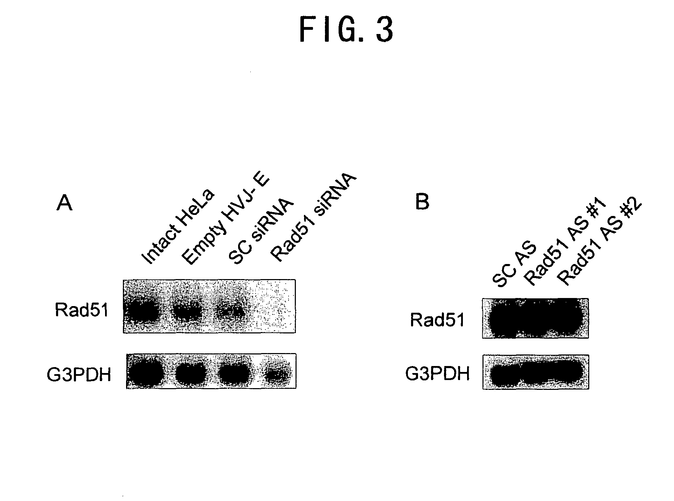

Fig. 3 represents the superiority of the siRNA to antisense oligonucleic acid. (A) sets forth the results of northern blots using HeLa cells in which Rad51 siRNA (321), siRNA having the scrambled sequence (SEQ ID NO: 6) (SC siRNA), or the viral vector alone (Empty HVJ-E) were introduced, respectively. (B) sets forth the results of northern blots using HeLa cells in which a conventional antisense oligonucleic acid against Rad51 (Rad51 AS#1 or Rad51 AS#2) or a scrambled Rad51 antisense oligonucleic acid (SC AS) were introduced.

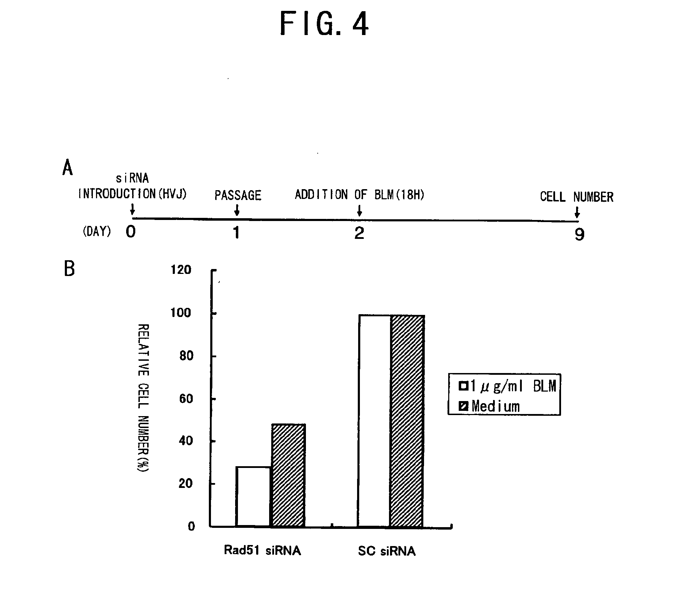

Fig. 4 depicts the antitumor effect of a combined use of Rad51 siRNA and bleomycin.

(A) sets forth the experimental schedule. (B) is a graph showing the number of surviving cancer cells in which Rad51 siRNA (target 321) (Rad51 siRNA) or a control scrambled siRNA (SC siRNA) was introduced using the HVJ envelope vector. Introduction of Rad51 siRNA (321) enhanced the effect of the anticancer agent by approximately 2-fold.

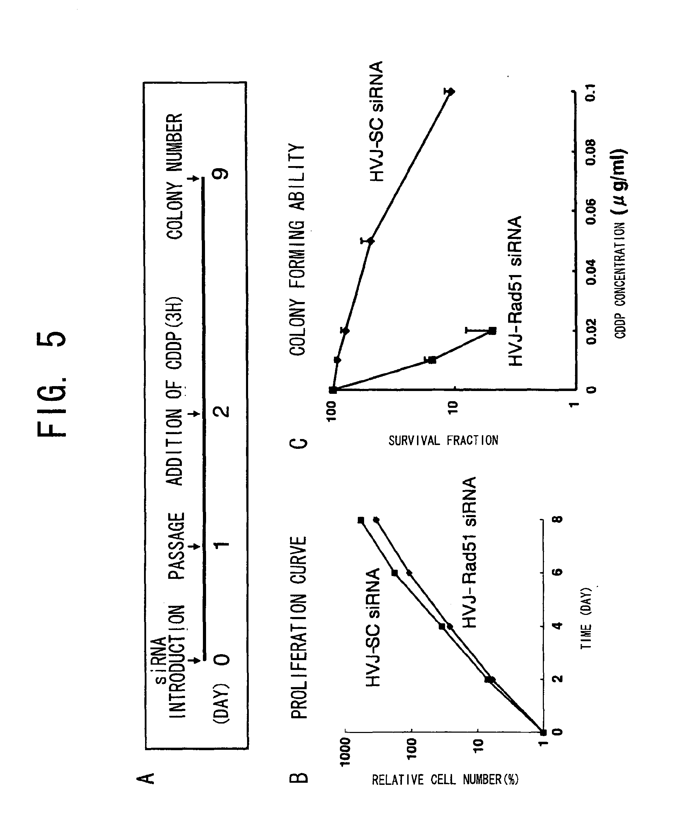

Fig. 5 depicts the enhanced sensitivity of cancer cells to cisplatin with Rad51 siRNA. (A) sets forth the experimental schedule. (B) is a graph showing the cell growth curve obtained by monitoring relative daily changes in cell number after introducing Rad51 siRNA (target 321) (HVJ-Rad51 siRNA) or control scrambled siRNA (HVJ-SC siRNA) into HeLa cells using the HVJ envelope vector. (C) is a graph showing the colony forming ability represented by the relative colony number after addition of cisplatin at the indicated concentrations. Combined use of Rad51 siRNA and cisplatin significantly inhibited the colony formation of cancer cells.

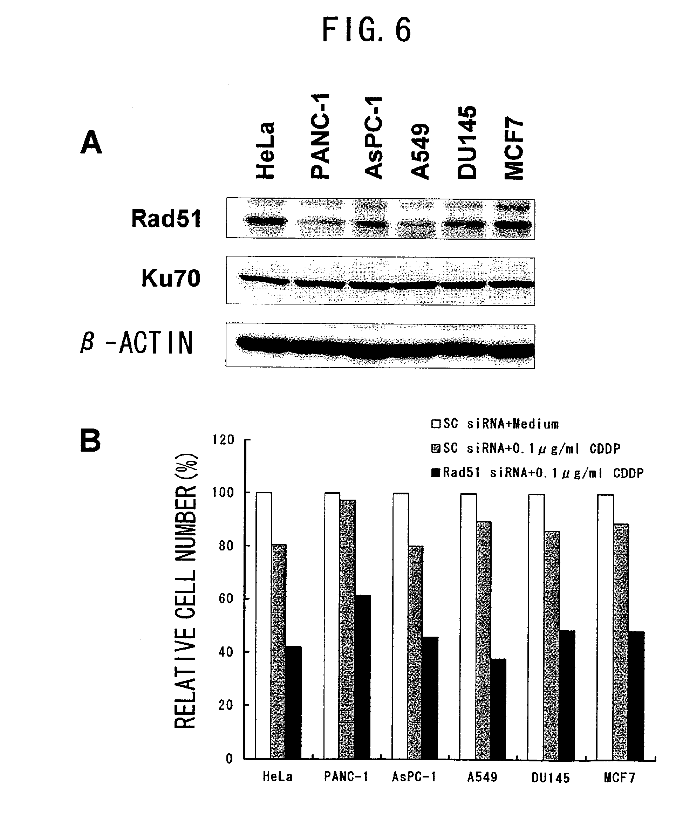

Fig. 6 includes a graph and a picture showing the enhanced cisplatin sensitivity of various human cancer cells resulting from the combined use of Rad51 siRNA (321) and cisplatin (CDDP). (A) depicts protein levels in various human cancer cells: PANC-1 (pancreatic cancer), AsPC-1 (pancreatic cancer), A549 (lung cancer), DU145 (prostate cancer), MCF7 (mammary carcinoma), and HeLa S-3 (cervical cancer). While the level of Ku70 and β-actin used as internal standards are almost the same, the protein level of Rad51 varies. (B) is a graph showing the survival rate of the cells to which CDDP (0.1 µg/ml) was administered in addition to siRNA. 80% or more cells survived using the control scrambled siRNA (SC siRNA + 0.1 µg/ml CDDP), whereas sensitivity to CDDP increased by 30% or more in all cancer cells using Rad51 siRNA (321) (Rad51 siRNA + 0.1 µg/ml CDDP).

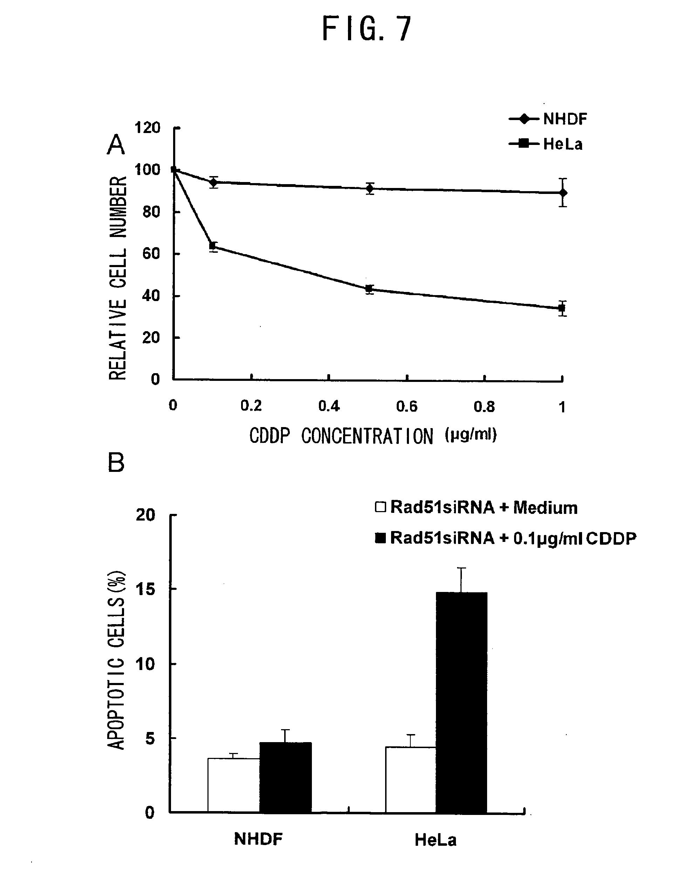

Fig. 7 is a graph showing changes in sensitivity to cisplatin (CDDP) when Rad51 siRNA (321) was introduced into normal human cells or cancer cells. (A) is a graph showing the cisplatin concentration dependency of normal human diploid fibroblasts (NHDF) or HeLa cells in which Rad51 siRNA (321) has been introduced. In the case of NHDF, no change in sensitivity was observed in response to increase in the cisplatin concentration where about 90% of cells survived, but the sensitivity increased in the HeLa cells depending on the CDDP concentration. (B) is a graph showing differences in apoptosis rate of Rad51 siRNA (321)-introduced NHDF or HeLa cells in the presence or absence of CDDP, determined as a percentage of Annexin V-positive cells. In the absence of CDDP (Rad51 siRNA + Medium), no change was observed in the apoptosis rate of both HeLa cells (4.0 ±1.1%) and NHDF (3.2 ± 0.5%). On the other hand, in the presence of CDDP (Rad51 siRNA + 0.1 µg/ml CDDP), the apoptosis rate of HeLa cells increased to 15.0%, whereas little increase was detected in NHDF (4.9%).

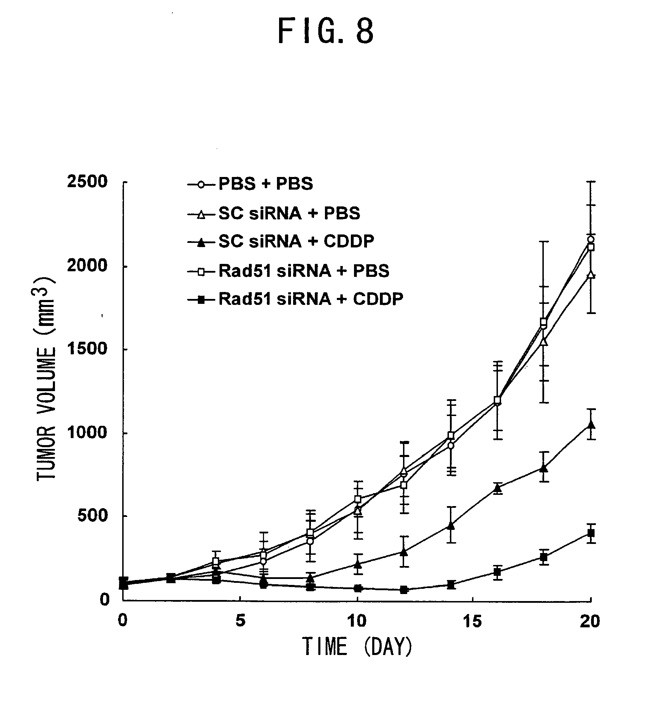

Fig. 8 is a graph showing the *in vivo* antitumor effect of the combined use of Rad51 siRNA (321) and cisplatin. Rad51 siRNA (target 321) (Rad51 siRNA) or the scrambled siRNA (SC siRNA) as a control was introduced into tumor masses formed by grafting HeLa cells onto a mouse, using the HVJ envelope vector, followed by intraperitoneal injection of cisplatin (CDDP), and then changes in tumor volume were monitored. The graph shows that combined use of Rad51 siRNA (321) and cisplatin can completely inhibit the tumor growth.

### Best Mode for Carrying Out the Invention

The present invention relates to double stranded RNA composed of a sense strand containing the nucleotide sequence of SEQ ID NO: 1 and a complementary strand thereof, wherein the length of the sense strand is 100 bases or less. The length of the sense strand constituting the double stranded RNA of the present invention is at least 19 bases (SEQ ID NO: 1), generally 19 to 50 bases, for example, 19 to 30 bases, preferably 19 to 25 bases, or 19 to 23 bases. The nucleotide sequence of the sense strand that constitutes the double stranded RNA of the present invention includes sequences that are not completely identical to the nucleotide sequence that constitutes the human Rad51 mRNA, so long as the double stranded RNA has the ability to suppress the Rad51 expression. For example, 1 to 5 nucleotide mutations are acceptable. More specifically, one nucleotide mutation in SEQ ID NO: 1 is acceptable.

When one wishes to make the sense strand of the double stranded RNA of the present invention longer than SEQ ID NO: 1, any nucleotide sequence may be added to the sequence of SEQ ID NO: 1. In the present invention, the nucleotide sequence to be added to the sequence of SEQ ID NO: 1 is preferably selected from the nucleotide sequence of human Rad51 mRNA. The nucleotide sequence of SEQ ID NO: 1 corresponds to the region of nucleotides 321 to 339 within the nucleotide sequence of human Rad51 mRNA. Thus, a contiguous nucleotide sequence containing this region is a preferred nucleotide sequence to be added in the context of the present invention.

In addition, the antisense strand that constitutes the double stranded RNA of the present invention is composed of a nucleotide sequence having a high complementarity to the sense strand. Herein, the term "high complementarity" means a complementarity of, for example, 90% or more, generally 95% or more, preferably 98% or more, and more preferably 99% or more. The complementarity of nucleotide sequences can be determined by a known algorithm, such as blastn. The nucleotide sequence of the antisense strand that constitutes the double stranded RNA of the present invention includes nucleotide sequences that are not completely complementary to the sense strand so long as the double stranded RNA has the ability to suppress the Rad51 expression. For example, 1 to 5 nucleotide mutations are acceptable. More specifically, one nucleotide mutation in the antisense strand is acceptable.

In the context of the present invention, the term "double stranded RNA" is defined as RNA having an accompanying complementary strand through base pairing. The sense and antisense strands of the double stranded RNA may be different molecules or the same molecule. When they are the same molecule, nucleotide sequences complementary to each other are placed on the same RNA molecule. Such molecules generally constitute a stem-loop structure due to base pairing between the sense and antisense strands. Specifically, the double stranded RNA of the present invention includes stem-loop RNA having a double stranded RNA structure as a partial structure.

The double stranded RNA of the present invention may be obtained through an enzymatic RNA synthesis utilizing RNA polymerase. Methods for such enzymatic RNA synthesis are known and carried out as follows. First, template DNA, DNA encoding RNA of interest placed downstream of an RNA polymerase-recognition sequence (promoter), is synthesized. When the sense and antisense strands of the double stranded RNA are to be expressed as different molecules, sense- and antisense-coding DNAs are designed to be expressed separately under the control of a promoter. The sense and antisense strands may be placed on the same vector, or incorporated into different vectors followed by co-transformation of the same cell. Alternatively, in order to express the both strands as a same molecule, DNA encoding a desired RNA constituting the stem-loop structure may be incorporated into a vector. In the RNA having the stem-loop structure, the nucleotide sequence of the loop is not particularly limited. Typically, nucleotide sequences of about 5 to 20 bases are used. For example, the nucleotide sequence 5'-ugugcuguc-3' (9 bases) can provide effective stem-loop siRNA.

Next, RNA polymerase is allowed to react with the template DNA in the presence of ribonucleotide substrates, and the nucleotide sequence of the template DNA is transcribed into RNA. RNA polymerase may be reacted with the template DNA in cells or cell-free systems. T7 RNA polymerase and the like may be used for the RNA transcription. The sense and antisense strands transcribed in cell-free systems are hybridized to constitute a double stranded RNA.

In general, to synthesize RNA of interest within cells, an expression vector in which a DNA encoding the RNA of interest has been incorporated is introduced into cells. Vectors for expressing siRNA in cells are known in the art. For example, expression vectors containing U6 promoter, H1 promoter, or the like, have been used as the siRNA expression vector using the PolIII transcription system, as described in the following references:
Brummelkamp et al., Science, 296: 550-553, 2002;
Lee et al., Nature Biotechnol., 20: 500-505, 2002;
Miyagishi et al., Nature Biotechnol., 20: 497-500, 2002;
Paddison et al., Genes & Dev., 16: 948-958, 2002;
Sui et al., Proc. Natl. Acad. Sci. USA, 99: 5515-5520, 2002;
Paul et al., Nature Biotechnol., 20: 505-508, 2002;
Yu et al., Proc. Natl. Acad. Sci. USA, 99: 6047-6052, 2002;
Tuschl et al., Nature Biotechnol., 20: 446-448, 2002;
McManus et al., Nature Rev. Genet., 3: 737-747, 2002; and
Dykxhoom et al., Nature Rev. Mol. Cell Biol., 77: 7174-7181, 2003.
The RNA transcribed by the PolIII transcription system has four Us added to the 3'-end of the transcript.

The following commercially available vectors (Takara Bio. Inc) may also be used as the siRNA expression vectors utilizing the PolIII transcription system:
siRNA expression vectors with U6 promoter:
   pBAsi-hU6 DNA: human U6 promoter (Accession X07425), and
   pBAsi-mU6 DNA: mouse U6 promoter (Accession X06980). siRNA expression vector with H1 promoter:
      pBAsi-hH1 DNA: human H1 promoter (Accession S68670).

In addition, the following viral vectors have been used to introduce siRNA constructs into cells to achieve long-term intracellular siRNA expression.
- Adenovirus (Zender et al., Proc. Natl. Acad. Sci. USA, 100: 7797-7802, 2003; Xia. et al., Nature Biotechnol., 20: 1006-1010, 2002);
- Lentivirus (Qin et al., Proc. Natl. Acad. Sci. USA, 100: 183-188, 2003);
- Retrovirus (Barton et al., Proc. Natl. Acad. Sci. USA, 99: 14943-14945, 2002); and
- Adeno-associated virus (Nature Med., 9: 1539-1544, 2003). These viral vectors may also be used as vectors for expressing the double stranded RNA of the present invention.
   RNA may also be chemically synthesized by a method known in the art.
   The double stranded RNA of the present invention suppresses Rad51 expression in cells *via* the RNAi effect. Namely, the double stranded RNA of the prevent invention is useful as siRNA to regulate Rad51 expression. Specifically, the present invention provides inhibitors of expression of the human Rad51 gene composed of a double stranded RNA having a sense strand containing the nucleotide sequence of SEQ ID NO: 1 and a complementary strand thereof (antisense strand), in which the sense strand is 100 bases or less in length, or a vector for expressing the double stranded RNA. The present invention also relates to a method for suppressing Rad51 expression including the step of administering the double stranded RNA composed of the sense strand having the nucleotide sequence of SEQ ID NO: 1 and complementary strand thereof, in which the sense strand is 100 bases or less in length, or a vector for expressing the double stranded RNA.
   An experiment using an antisense nucleotide has shown that suppression of Rad51 expression in cancer cells enhances the radiosensitivity of the cells. The double stranded RNA of the present invention suppresses Rad51 expression more strongly than the antisense nucleotide. Thus, the double stranded RNA of the present invention to treat proliferative disorders *via* suppression of Rad51 expression. Accordingly, the present invention relates to pharmaceutical compositions that include as an active ingredient a double stranded RNA composed of a sense strand having the nucleotide sequence of SEQ ID NO: 1 and a complementary strand thereof, or a vector capable of expressing the double stranded RNA.
   The inventors of the present invention further found that the double stranded RNA of the present invention enhances the therapeutic effects of chemotherapeutic agents. Accordingly, the present invention provides pharmaceutical compositions including as active ingredients both of the double stranded RNA composed of the sense strand having the nucleotide sequence of SEQ ID NO: 1 and complementary strand thereof, or the vector capable of expressing the double stranded RNA, and a chemotherapeutic agent.
   The pharmaceutical composition of the present invention may also be provided as a combined composition of the pharmaceutical composition that includes the double stranded RNA composed of the sense strand having the nucleotide sequence of SEQ ID NO: 1 and complementary strand thereof, or a vector expressing the double stranded RNA, and a pharmaceutical composition that includes the chemotherapeutic agent as the active ingredients, each of which has been prepared separately. The pharmaceutical composition of the present invention is particularly useful for treating cellular proliferative disorders. Herein, cellular proliferative disorders include, for example, cancer.
   The chemotherapeutic agents herein include carcinostatic agents, anticancer agents, and antitumor agents or antineoplastic agents. In the context of the present invention, preferred chemotherapeutic agents are those having either a nucleic acid synthesis inhibitory activity or a nucleic acid-impairing activity, or both. In the present invention, the term nucleic acid synthesis inhibitory activity refers to an ability to inhibit DNA or RNA synthesis. The nucleic acid-impairing activity includes activities to modify or cleave nucleic acids. Specifically, the chemotherapeutic agents detailed below are preferred. Among those chemotherapeutic agents, bleomycin and cisplatin can produce a composition that is particularly effective in terms of cytotoxicity to cancer cells, when combined with the double stranded RNA. Thus, bleomycin and cisplatin are preferred chemotherapeutic agents to be used in the present invention.

### Bleomycins:

Bleomycins are a group of antibiotics obtained from *Streptomyces verticillus.* Structurally, they are water-soluble, basic sugar peptide compounds. Bleomycin A2, bleomycin B2, peplomycin, and the like have been practically applied as carcinostatic agents. They have a DNA molecule-cleaving activity and an ATP dependent DNA ligase-inhibitory activity. They are used for treating squamous carcinoma and the like. The pharmaceutically acceptable salts of bleomycins include, for example, bleomycin hydrochloride, bleomycin sulfate, and peplomycin sulfate.

### Anthraquinone series carcinostatic agents:

These are products of *Streptomyces,* many of which have therapeutic effects particularly against acute leukemia. Doxorubicin, which is synthesized by chemically modifying the daunorubicin structure, has been shown to have a strong pharmacological activity against solid tumors. It has been known that many mechanisms are involved in the pharmacological activity of the anthraquinone series carcinostatic agents. Specifically, intercalation into DNA, interaction with topoisomerase II, free radical production, and action on cell membrane have been reported. Mitoxantrone is used for treating myelocytic leukemia and breast cancer. Idarubicin can be administered *via* an oral route.
- mitoxantrone hydrochloride
- aclarubicin hydrochloride
- idarubicin hydrochloride

### Mitomycins:

Mitomycins are antibiotics isolated from *Streptomyces caespitosus* and derivatives thereof. They have three carcinostatic structures: quinone, urethane, and aziridine, within their molecules. They inhibit DNA synthesis by binding to the side chain of DNA *via* DNA alkylation. They are known to have a wide spectrum of action and be effective against a variety of cancers.

### Actinomycins:

Actinomycins include compounds isolated from *Streptomyces* and derivatives thereof. For example, actinomycin D isolated from *Streptomyces parvullus* is a macromolecule having a connected structure of an orange-yellow chromophore and a peptide. Binding of actinomycin D to a guanine in the double helix structure of DNA results in transcriptional inhibition. They are commonly used for treating Wilms' tumor, Hodgkin's disease, and the like.

### Camptothecins:

Camptothecins are plant alkaloids extracted from *Nothapodytes foetida, Camptotheca acuminata,* and the like, and analogues thereof. They have a quinoline backbone in their molecular structure. Irinotecan (topotecin), nogitecan, and exatecan are derivatives of plant camptothecin.

### Cisplatins:

Cisplatins are complex compounds containing platinum in their structure and inhibit cancer development. This group of carcinostatic agents includes, for example, carboplatin and nedaplatin. These compounds are thought to exhibit carcinostatic activity through binding to guanine moieties of DNA. Cisplatin, paraplatin, briplatin, and the like are known.

### Streptozotocin:

Streptozotocin has been confirmed to be clinically useful as an anticancer agent, despite the fact that it shows direct toxicity to pancreatic islet cells as methylnitrosourea. Streptozotocin is applicable to therapeutics for pancreatic insulinoma or gastrointestinal endocrine tumor.

### 5-fluorouracil (5-FU) and derivatives thereof:

The following compounds are known as carcinostatic agents classified into antipyrimidines:
- 5-fluorouracil (5-FU);
- 5-fluorodeoxyuridine (FUDR);
- tegafur;
- cytarabine;
- ancitabine; and
- azauridine.
These compounds inhibit DNA polymerase through antagonizing pyrimidine during DNA synthesis. These compounds have been confirmed to be effective against a variety of solid tumors and leukemia.

### Pirarubicin:

During the screening of microorganisms (actinomycetes) in culture in search of a new anthracycline antibiotic with low cardiotoxicity, aclarubicin (aclacinomycin A) was found in 1975. Pirarubicin, a less cardiotoxic adriamycin derivative obtained from further investigations for the synthesis of adriamycin derivatives, is now used as a cancer chemotherapeutic agent.

According to the findings of the present inventors, inhibition of Rad51 expression enhanced the anticancer activity of the chemotherapeutic agents bleomycin and cisplatin. In other words, Rad51 inhibitors enhanced the anticancer activity of bleomycin or cisplatin. Accordingly, the present invention provides pharmaceutical compositions composed of bleomycin or a pharmaceutically acceptable salt thereof, or cisplatin or a pharmaceutically acceptable salt thereof, and a Rad51 inhibitor. The present invention also relates to uses of bleomycin or a pharmaceutically acceptable salt thereof, or cisplatin or a pharmaceutically acceptable salt thereof, and a Rad51 inhibitor, in the production of the pharmaceutical composition for treating cellular proliferative disorders. In addition, the present invention provides a method for treating cellular proliferative disorders including the step of administering bleomycin or a pharmaceutically acceptable salt thereof, or cisplatin or a pharmaceutically acceptable salt thereof, and a Rad51 inhibitor. Preferred cellular proliferative disorders to be treated in the present invention are cancers.

In the present invention, the Rad51 inhibitor includes any compound capable of inhibiting either Rad51 expression or an activity thereof, or both. A preferred Rad51 inhibitor is siRNA. For example, the double stranded RNA containing the nucleotide sequence as shown in SEQ ID NO: 1 as the sense strand strongly inhibits Rad51 expression. In addition, an antisense nucleic acid that inhibits Rad51 expression is known in the art (US 2003/0229004).

The double stranded RNA of the present invention or the DNA capable of expressing the double stranded RNA can be used for cancer therapy by introducing it into cancer cells. Any method may be used for introducing it into the cancer cells. For example, a variety of reagents for transfection are well known in the art. Alternatively, the double stranded RNA may be introduced into cancer cells by encapsulating it into a liposome or a viral envelope vector.

The pharmaceutical composition of the present invention composed of the double stranded RNA and the chemotherapeutic agent may also be introduced into cancer cells in a similar manner and used for cancer treatment. For example, a liposome and a viral envelope vector are preferable as a carrier for introducing into cancer cells the pharmaceutical composition of the present invention composed of the double stranded RNA and the chemotherapeutic agent.

For example, an *in vivo* gene transfer method utilizing the liposome in combination with viral envelope and the HVJ-liposome-mediated gene transfer method have been developed (Science, 243: 375-378 (1989); Anal. NYAcad. Sci. 772: 126-139 (1995)). These methods have been successfully used for *in vivo* gene transfer into a variety of tissues including liver, kidney, vascular wall, heart, and brain (Gene Therapy 7: 417-427 (2000); Science 243: 375-378 (1989); Biochem. Biophys. Res. Commun. 186: 129-134 (1992); Proc. Natl. Acad. Sci. USA 90: 8474-8478 (1993); Am. J. Physiol. 271 (Regulatory Integrative Comp. Physiol.40): R1212-R1220(1996)).

However, in the HVJ-liposome method, the procedure is complicated because two different vehicles, a virus and a liposome, must be prepared. In addition, the average diameter of the fusion product of HVJ with the liposome reaches 1.3-fold larger than that of the viral particle. As a result, the cell fusion activity decreases to 10% or less of that of wild type virus, and in certain tissues, no or little gene can be introduced. Accordingly, a method of gene transfer using a viral envelope vector has been developed as the method enabling a safer and more efficient gene therapy (Japanese Patent Application *Kokai* Publication No. (JP-A) 2001-286282). In this method, a gene or the like is encapsulated in a viral envelope of an inactivated virus which cannot replicate viral proteins, and then the resulting virus can be used as a vector for gene transfer into cultured cells, living tissues, or the like. It is known that use of such a viral envelope vector enables the safe and highly efficient introduction of genes into liver, skeletal muscles, uterus, brain, eyes, carotid arteries, skin, blood vessels, lungs, heart, kidneys, spleen, cancerous tissues, nerves, B lymphocytes, respiratory tissues, suspended cells, or the like.

The viral envelope may be prepared by mixing a desired pharmaceutical composition with the purified virus in the presence of a detergent, or freezing and thawing a mixture of the virus and the pharmaceutical composition (JP-A 2001-286282). The pharmaceutical composition as used herein includes a pharmaceutical composition composed of the double stranded RNA, a DNA expressing the double stranded RNA, or the double stranded RNA and a chemotherapeutic agent.

The virus useful for the viral-envelope method includes, for example, those belonging to the families of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae. More specifically, Sendai virus (HVJ), retrovirus, adenovirus, adeno-associated virus, herpes virus, vaccinia virus, pox virus, or influenza virus may be used. Both of wild type and recombinant type viruses may be used. In particular, as for HVJ, the recombinant type HVJ may also be used as reported by Hasan M. K. et al. (J. General Virol. 78: 2813-2820 (1997)) or Yonemitsu Y. et al. (Nature Biotech. 18: 970-973 (2000)).

In general, strain Z (available from ATCC) is preferable as HVJ used for the HVJ-liposome method and the HVJ-envelope method, but essentially any other HVJ strain (for example, ATCC VR-907 and ATCC VR-105) may also be used. In addition, during the preparation of the viral envelope, a desired expression vector may be mixed after inactivation of the purified virus by UV irradiation or the like. Detergents for use in mixing a virus and a pharmaceutical composition include, for example, octyl glucoside, Triton X-100, CHAPS, and NP-40. The viral envelope vector thus prepared may be introduced into the targeted tissue to be treated or prevented by injection or the like. In addition, the vector may be stored in a frozen state at -20°C for at least 2 to 3 months.

The methods for introducing the viral envelope vector containing the pharmaceutical composition of the present invention into a patient include an *in vivo* method in which an agent for gene therapy is delivered directly inside the body, and an *ex vivo* method in which the agent for gene therapy is introduced into certain cells taken out of a human body, and then the cells are returned inside the body (NIKKEI SCIENSE, April: 20-45 (1994); The Pharmaceuticals Monthly, 36 (1): 23-48 (1994); Experimental Medicine Extra number, 12 (15) (1994); The Japan Society of Gene Therapy. (ed.), "Handbook of gene therapy development research", NTS Inc. (1999)). In the present invention, *the in vivo* method is particularly preferable.

The dosage form may vary depending on the administration route as described above, and includes various known forms (for example, liquid forms). For example, when formulating an injection containing the double stranded RNA or the double stranded RNA and the chemotherapeutic agent as active ingredients, such injections may be prepared according to conventional methods. For example, the components may be dissolved in an appropriate solvent (e.g., a buffer solution such as PBS, physiological saline, and sterile water), sterilized through filtration if necessary, and then packed in a sterile container. Conventional carriers may also be added to the injection as appropriate. In addition, liposome preparations such as HVJ-liposome can be in the form of a suspension, a frozen preparation, and a centrifugally concentrated frozen preparation.

An appropriate administration method and site for the pharmaceutical composition of the present invention may be chosen depending on the conditions of cancer to be treated. A parenteral administration is particularly preferable. Dosage of the double stranded RNA of the present invention usually ranges from 0.5 to 100 mmol/tumor, for example, 1 to 50 mmol/tumor. In addition, when administering a DNA to express the double stranded RNA, the dosage ranges usually from 1 to 1000 µg/tumor, for example, 10 to 500 µg/tumor. When used in combination with a chemotherapeutic agent, a standard administration schedule for the chemotherapeutic agent may be employed. For example, when combined with bleomycin as the chemotherapeutic agent, the dosage ranges usually from 0.1 to 100 mg (potency)/day per adult (60 kg body weight), for example, 1 to 50 mg (potency)/day, preferably 5 to 30 mg (potency)/day. When combined with cisplatin, the dosage ranges usually from 1 to 1000 mg/m² (body surface area) per adult (60 kg body weight), for example, 5 to 500 mg/m² (body surface area), preferably 10 to 100 mg/m² (body surface area).

An exemplary administration schedule used in the present invention is as follows. When bleomycin is used as the chemotherapeutic agent, a dosage of 15 to 30 mg (in the case of intravenous injection, intramuscular injection, or subcutaneous injection), or 5 to 15 mg (intraarterial injection) may be administered once a day, basically twice a week. The number of administrations may be changed to once a week, observing the time course of patient's conditions.

When cisplatin is used as the chemotherapeutic agent, a dosage of 15 to 20 mg/m² (body surface area) may be administered for 5 days (once a day), and, after a two-week rest, the same schedule may be repeated (repeated administration). The schedule for the repeated administration may be changed as needed. For example, a dose of 70 to 90 mg/m² (body surface area) may be administered for 5 days (once a day), and after a three-week rest, the same schedule may be repeated (repeated administration).

All prior art documents cited herein are incorporated by reference in their entirety.

### Examples

The present invention is described in more detail below with reference to the following

### Examples:

### [Example 1] Preparation of Rad51 siRNA and introduction into cells

Human Rad51 (Accession number NM_002875) siRNAs were prepared by Dharmacon, USA, each containing one of the following five sense strands and its complementary antisense strand (Fig. 1).
target 321: GAGCUUGACAAACUACUUC (SEQ ID NO: 1)
target 462: GGAAAGGCCAUGUACAUUG (SEQ ID NO: 2)
target 989: UGCAGAUGGAGUGGGAGAU (SEQ ID NO: 3)
target 89: GUGUGGCAUAAAUGCCAAC (SEQ ID NO: 4)
target 828: GCGAUGUUUGCUGCUGAUC (SEQ ID NO: 5)

These five kinds of siRNAs were introduced into HeLa cells (ATCC. CCL-2; Gey, G. O. et al., Cancer Res., 12: 264-265, 1952) using the HVJ envelope vector.

The Rad51 siRNAs were introduced as follows. One day before the transfection of siRNA, 1x10⁵ HeLa cells were plated onto a 6-well plate.

According to a known method (Kaneda Y. et al., Mol Ther., 2002 Aug; 6 (2): 219-26), an inactivated HVJ was prepared. To a sample tube containing 6000 HAU (hemagglutinating units) inactivated HVJ, 60 µl of 40 µM Rad51 siRNA (containing one of SEQ ID NOs: 1 to 5) or a control siRNA (SEQ ID NO: 6) containing the scrambled sequence (5'-gcgcgcuuuguaggauucg-3') was added, followed by 6 µl of 2% Triton X-100. After centrifugation at 10000x g for 10 min, followed by removal of supernatant, the precipitate was suspended in 180 µl of PBS (phosphate buffered saline). 15 µl of 10 mg/ml protamine sulfate was added (at the final concentration of 50 µg/ml), followed by 3 ml of DMEM (Dulbecco's Modified Eagle Medium). 500 µl (1000 HAU) of the solution containing the HVJ envelope vector and each of siRNAs was added to HeLa cells in each well, followed by incubation at 37°C for 30 min. After that, the HVJ envelope vector solution was removed, and then the cells were incubated at 37°C under 5% CO₂.

Northern blot analysis of Rad51 mRNA performed one day after the introduction of siRNA revealed that one of the siRNAs showed an inhibitory activity against Rad51 expression, which has the sequence GAGCUUGACAAACUACUUC (SEQ ID NO: 1) corresponding to the sequence of 19 bases (with +2 nucleotide overhang) starting from codon 321 (Fig. 1; hereafter also referred to as "321"). Other 4 candidates (SEQ ID NO: 2 to 5) did not show any inhibitory activity. Accordingly, the Rad51 siRNA (321, SEQ ID NO: 1) that showed the inhibitory activity was used for the subsequent experiments.

### [Example 2] Suppression of the Rad51 protein expression by siRNA

The Rad51 siRNA (321, SEQ ID NO: 1) that showed the inhibitory activity in Example 1 was introduced into HeLa cells as described in Example 1 and the inhibitory activity against the Rad51 protein expression was monitored on a day-by-day basis. As a control, siRNA having the scrambled sequence (SEQ ID NO: 6) was introduced into HeLa cells in the same manner, and the Rad51 protein expression was monitored on a day-by-day basis. Rad51 siRNA (321) inhibited the Rad51 protein expression for 4 days as completely as the expression level reached the detection limit or below. The expression level on day 5 was 10% or less of that before the introduction (Fig. 2).

### [Example 3] Comparison of inhibitory activity against Rad51 protein expression between siRNA and antisense nucleic acid

According to the reference (Ohnishi, T., Taki, T., Hiraga, S., Arita, N., Morita, T., In vitro and in vivo potentiation of radiosensitivity of malignant gliomas by antisense inhibition of the Rad51 gene., Biochem. Biophys. Res. Comm., 1998; 245: 319-324), 15 bp Rad51 AS#1 (5' ggcttcactaattcc 3') and Rad51 AS#2 (5' cgtatgacagatctg 3') were prepared as Rad 51 antisense oligonucleic acids. Rad51 AS#1 and Rad51 AS#2 correspond to the nucleotides 368 to 382 and the nucleotides 578 to 592 of the Rad51 gene, respectively.

Approximately 80 pmol of each of Rad51 siRNA (321), Rad51 AS#1, and Rad51 AS#2 was introduced into 1x 10⁵ HeLa cells as described in Example 1. As controls, siRNA (SC siRNA) having the scrambled sequence (SEQ ID NO: 6), the viral vector alone (Empty HVJ-E), or a scrambled sequence (SC AS, 5' tcgcgatcaccttat 3') with respect to the Rad51 antisense oligonucleic acid was introduced into HeLa cells in the same manner. G3PDH (glyceraldehyde-3-phosphate dehydrogenase) was used as an internal standard.

One day later, the inhibitory effect on Rad51 protein expression was assessed by northern blot analysis. Rad51 mRNA expression was so strongly inhibited in HeLa cells in which Rad51 siRNA (321) was introduced that the expression was undetectable by northern blot analysis (Fig. 3A). In contrast, Rad51 antisense oligonucleic acids (Rad51 AS#1 and Rad51 AS#2) had low effects (Fig. 3B), indicating the superiority of siRNA to antisense oligonucleic acid.

### [Example 4] Enhancement of anticancer activity of Rad51 siRNA by a combined use with bleomycin

Rad51 siRNA (321) and the control scrambled siRNA (SEQ ID NO: 6) were separately introduced into HeLa cells using the HVJ envelope vector as described in Example 1, and two days later, treated with the anticancer agent bleomycin (BLM, 1 µg/ml) for 18 hours (Fig. 4A). With bleomycin (BLM) treatment, the anticancer effect was approximately 2-fold enhanced as compared with that without the bleomycin treatment (Fig. 4B).

### [Example 5] Enhancement of anticancer activity of Rad51 siRNA by a combined use with cisplatin

The inventors of the present invention found that Rad51 is associated with repair of double strand cleavage by cisplatin (cis-diamminedichloroplatinum (II); CDDP). The inventors of the present invention ascertained that the Rad51 level increases approximately two times when CDDP is added to cancer cells, and cancer cells become resistant to CDDP when Rad51 gene is overexpressed. These findings suggest that inhibition of Rad51 expression reduces the cellular resistance to CDDP. Accordingly, Rad51 siRNA (321) or the control scrambled sequence (SEQ ID NO: 6) was introduced into HeLa cells, cancer cells expressing Rad51 at a high level, to monitor changes in the relative cell number over time. Two days after the introduction, the cells were treated with cisplatin (CDDP) for 3 hours, and the number of colonies was counted on day 9 (Fig. 5A). Proliferative ability of the cells in which Rad51 siRNA (321) was introduced slightly reduced as compared with control (Fig. 5B). In contrast, as shown in Fig. 5C, when two days after Rad51 siRNA (321) introduction, the cisplatin (CDDP) treatment was performed at the indicated concentrations for 3 hours, colony-forming ability of the Rad51 siRNA (321)-introduced group strikingly declined compared with that of the control scrambled sequence-introduced group, indicating that colony forming ability of cancer cells was significantly suppressed.

### [Example 6] Enhancement of anticancer activity in various human cancer cells by the combined use of Rad51 siRNA and cisplatin

Rad51 siRNA (321) or the scramble siRNA (SEQ ID NO: 6) was introduced into various human cancer cells, PANC-1 (pancreatic cancer), AsPC-1 (pancreatic cancer), A549 (lung cancer), DU145 (prostate cancer), MCF7 (mammary carcinoma), and HeLa S-3 (cervical cancer), using the HVJ envelope vector along with simultaneous cisplatin (CDDP) administration, and two days after, cell proliferation assay was performed. Ku70 and β-actin were used as internal standards. As a result of monitoring protein levels in each cell, Ku70 and β-actin levels were almost the same among those human cancer cells, but the Rad51 protein levels varied (Fig. 6A). When cisplatin (CDDP, 0.1 µg/ml) was administered simultaneously, 80% or more of the scrambled siRNA-introduced cancer cells survived, whereas survival rate of every cancer cell in which RAD51 siRNA (321) was introduced decreased, resulting in 30% or more increase in CDDP sensitivity (Fig. 6B). Thus, Rad51 siRNA (321) was also shown to have the enhancing effect on the cisplatin sensitivity of various human cancer cells.

### [Example 7] Comparison of cisplatin sensitivity between normal human cells and cancer cells

Rad51 siRNA (321) was introduced into normal human cells or cancer cells, using the HVJ envelope vector, to determine differences in CDDP sensitivity between them. When Rad51 siRNA (321) was introduced into normal human diploid fibroblasts (NHDF) as normal human cells, no change in sensitivity was observed in response to increase in cisplatin concentrations, and approximately 90% of cells survived. On the other hand, when HeLa cells were used as cancer cells, survival cells decreased in response to increase in cisplatin concentrations after introduction of Rad51 siRNA (321), indicating that the sensitivity is enhanced in a CDDP concentration-dependent manner (Fig. 7A). Accordingly, apoptosis rates of NHDF and HeLa cells after the introduction of Rad51 siRNA (321) were determined as a percentage of Annexin V-positive cells in the presence or absence of 0.1 µg/ml CDDP (Fig. 7B). In the absence of CDDP, no difference in the apoptotic cell rate was observed between HeLa cells (4.0 ± 1.1%) and NHDF (3.2 ± 0.5%). In contrast, in the presence of CDDP, the apoptosis rate increased to 15.0% in HeLa cells, whereas it scarcely increased (4.9%) in NHDF. These data showed that increased CDDP sensitivity is a phenomenon specifically observed in cancer cells, allowing a highly safe treatment with Rad51 siRNA (321).

### [Example 8] Enhancement of the cancer-treating effect of Rad51 siRNA

HeLa cells were grafted onto a nude mouse to develop tumor masses. First, Rad51 siRNA (321) was introduced into the tumor masses, and two days later, 200 µg of CDDP was intraperitoneally injected and siRNA (321) was re-injected into the tumor masses simultaneously. Two days after that, an additional injection of Rad51 siRNA (321) into the tumor masses was carried out. The tumor proliferation-inhibitory activity was very weak by the injection of Rad51 siRNA (321) alone and the tumor growth was only partially inhibited by the combined use of the scrambled siRNA and CDDP. In contrast, the combined use of Rad51 siRNA (321) and CDDP almost completely inhibited the tumor growth, showing a significant carcinostatic activity (Fig. 8). Introducing efficiency of Rad51 siRNA into the tumor masses was 40 to 50%, when the siRNA was administered *via* the HVJ envelope vector.

### Industrial Applicability

The double stranded RNA of the present invention or the composition containing the same is useful for regulating Rad51 expression. It is possible to inhibit cancer cell proliferation or impair cancer cells themselves by regulating Rad51 expression. In addition, regulation of Rad51 expression is useful for enhancing radiosensitivity of cancer cells. Moreover, it is revealed that the double stranded RNA of the present invention exhibits a strong carcinostatic activity by a combined use with a chemotherapeutic agent. As described above, the present invention is useful for treating cancers.

## Claims

1. A double stranded RNA composed of a sense strand comprising the nucleotide sequence of SEQ ID NO: 1 and a complementary strand thereof, wherein said sense strand is 100 nucleotides or less in length.

2. A vector expressing a double stranded RNA composed of a sense strand comprising the nucleotide sequence of SEQ ID NO: 1 and a complementary strand thereof, wherein said sense strand is 100 nucleotides or less in length.

3. An inhibitor of the expression of the Rad51 gene comprising the double stranded RNA of claim 1 or the vector of claim 2 as an active ingredient.

4. The inhibitor of claim 3, wherein said Rad51 gene is a human Rad51 gene.

5. A pharmaceutical composition comprising a double stranded RNA composed of a sense strand comprising the nucleotide sequence of SEQ ID NO: 1 and a complementary strand thereof, or a vector capable of expressing the RNA, as an active ingredient.

6. A pharmaceutical composition comprising a double stranded RNA composed of a sense strand comprising the nucleotide sequence of SEQ ID NO: 1 and a complementary strand thereof, or a vector capable of expressing the RNA, and a chemotherapeutic agent as active ingredients.

7. The pharmaceutical composition of claim 6, wherein said pharmaceutical composition is used to treat a cancer or malignant tumor.

8. The pharmaceutical composition of claim 6, wherein said chemotherapeutic agent is selected from the group consisting of carcinostatic agents, anticancer agents, antitumor agents, and antineoplastic agents.

9. The pharmaceutical composition of claim 6, wherein said chemotherapeutic agent has either a nucleic acid synthesis inhibitory activity or a nucleic acid impairing activity, or both.

10. The pharmaceutical composition of claim 6, wherein said chemotherapeutic agent is at least one compound selected from the group consisting of bleomycins, anthraquinone series carcinostatic agents, mitomycins, actinomycins, camptothecins, cisplatins, streptozotocin, 5-fluorouracil (5-FU) and derivatives thereof, pirarubicin, and pharmaceutically acceptable salts thereof.

11. The pharmaceutical composition of claim 10, wherein said bleomycins comprise a compound selected from the group consisting of bleomycin and peplomycin.

12. The pharmaceutical composition of claim 10, wherein said pharmaceutically acceptable salts of bleomycins comprise a compound selected from the group consisting of bleomycin hydrochloride, bleomycin sulfate, and peplomycin sulfate.

13. The pharmaceutical composition of claim 10, wherein said cisplatins comprise a compound selected from the group consisting of cisplatin, paraplatin, and briplatin.

14. The pharmaceutical composition of claim 6, wherein said chemotherapeutic agent is encapsulated in a viral envelope vector together with the double stranded RNA.

15. The pharmaceutical composition of claim 14, wherein said viral envelope vector is composed of an envelope derived from a virus belong to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae.

16. The pharmaceutical composition of claim 14, wherein said viral envelope vector is composed of an envelope derived from a virus selected from the group consisting of Sendai virus, retrovirus, adenovirus, adeno-associated virus, Herpes virus, vaccinia virus, poxvirus, and influenza virus.

17. A combined composition comprising (a) a pharmaceutical composition comprising a double stranded RNA composed of a sense strand comprising the nucleotide sequence of SEQ ID NO: 1 and a complementary strand thereof as an active ingredient, and (b) a pharmaceutical composition comprising a chemotherapeutic agent as an active ingredient.

18. A combined composition comprising (a) a pharmaceutical composition comprising a vector capable of expressing a double stranded RNA composed of a sense strand comprising the nucleotide sequence of SEQ ID NO: 1 and a complementary strand thereof as an active ingredient, and (b) a pharmaceutical composition comprising a chemotherapeutic agent as an active ingredient.

19. The combined composition of claim 17 or 18, wherein said combined composition is used to treat a cancer or malignant tumor.

20. The combined composition of any one of claims 17 to 19, wherein said chemotherapeutic agent is selected from the group consisting of carcinostatic agents, anticancer agents, antitumor agents, and antineoplastic agents.

21. The combined composition of any one of claims 17 to 19, wherein said chemotherapeutic agent has either a nucleic acid synthesis inhibitory activity or a nucleic acid impairing activity, or both.

22. The combined composition of any one of claims 17 to 19, wherein said chemotherapeutic agent is at least one compound selected from the group consisting of bleomycins, anthraquinone series carcinostatic agents, mitomycins, actinomycins, camptothecins, cisplatins, streptozotocin, 5-fluorouracil (5-FU) and derivatives thereof, pirarubicin, and pharmaceutically acceptable salts thereof.

23. The combined composition of claim 22, wherein said bleomycins comprise a compound selected from the group consisting of bleomycin and peplomycin.

24. The combined composition of claim 22, wherein said pharmaceutically acceptable salts of bleomycins comprise a compound selected from the group consisting of bleomycin hydrochloride, bleomycin sulfate, and peplomycin sulfate.

25. The combined composition of claim 22, wherein said cisplatins comprise a compound selected from the group consisting of cisplatin, paraplatin, and briplatin.

26. The combined composition of claim 17 or 18, wherein said double stranded RNA is encapsulated in a viral envelope vector.

27. The combined composition of claim 26, wherein said viral envelope vector is composed of an envelope derived from a virus belong to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae, and Hepadnaviridae.

28. The combined composition of claim 26, wherein said viral envelope vector is composed of an envelope derived from a virus selected from the group consisting of Sendai virus, retrovirus, adenovirus, adeno-associated virus, Herpes virus, vaccinia virus, poxvirus, and influenza virus.

29. A method for inhibiting Rad51 expression, comprising the step of administering into cells the double stranded RNA of claim 1 or the vector of claim 2.

30. A method for treating a cancer or malignant tumor, comprising the step of administering a double stranded RNA composed of a sense strand comprising the nucleotide sequence of SEQ ID NO: 1 and a complementary strand thereof, or a vector capable of expressing the RNA, and a chemotherapeutic agent.

31. The method of claim 30, wherein said step of administering the double stranded RNA or the vector capable of expressing the RNA and the chemotherapeutic agent comprises administering a viral envelope vector in which the double stranded RNA and the chemotherapeutic agent have been encapsulated.

32. The method of claim 30, wherein said step of administering the double stranded RNA or the vector capable of expressing the RNA and the chemotherapeutic agent comprises separately administering a pharmaceutical composition comprising the double stranded RNA or the vector capable of expressing the RNA as an active ingredient, and a pharmaceutical composition comprising the chemotherapeutic agent as an active ingredient.

33. Use of the double stranded RNA of claim 1 or the vector of claim 2 for preparing a pharmaceutical composition for treating a cancer or malignant tumor.

34. Use of the double stranded RNA of claim 1 or the vector of claim 2, wherein said pharmaceutical composition comprises a chemotherapeutic agent.

35. A pharmaceutical composition comprising bleomycin or a pharmaceutically acceptable salt thereof and a Rad51 inhibitor.

36. A pharmaceutical composition comprising cisplatin or a pharmaceutically acceptable salt thereof and a Rad51 inhibitor.

37. A combined composition comprising (a) a pharmaceutical composition comprising bleomycin or a pharmaceutically acceptable salt thereof as an active ingredient, and (b) a pharmaceutical composition comprising a Rad51 inhibitor as an active ingredient.

38. A combined composition comprising (a) a pharmaceutical composition comprising cisplatin or a pharmaceutically acceptable salt thereof as an active ingredient, and (b) a pharmaceutical composition comprising a Rad51 inhibitor as an active ingredient.

39. The combined composition of claim 37 or 38, wherein said combined composition is intended to treat a cancer or malignant tumor.
